# EUROPÄISCHE PATENTSCHRIFT

(11) **EP 1 566 194 B1**
(45) Veröffentlichungstag und Bekanntmachung des Hinweises auf die Patenterteilung: **07.05.2008**
(21) Anmeldenummer: 05001546.0
(22) Anmeldetag: 26.01.2005
(51) Int. Cl.: A61M 5/315

(54) **Spritze, insbesondere für medizinische Anwendungen**
Syringe, in particular for medical applications
Seringue, en particulier pour usages médicaux

(30) Priorität: 20.02.2004 DE 102004009919
(43) Veröffentlichungstag der Anmeldung: 24.08.2005
(73) Patentinhaber: Schott AG, 55122 Mainz (DE)
(72) Erfinder: Wolbring, Peter, Dr., 66606 St. Wendel (DE); Koeppel, Sascha, 9326 Horn (CH)
(74) Vertreter: Duhme, Torsten

(56) Entgegenhaltungen:
- EP-A- 0 764 450
- WO-A-92/08507
- CH-A- 360 168
- DE-A1- 10 220 034

## Beschreibung

Die vorliegende Erfindung betrifft eine Spritze, insbesondere für medizinische Anwendungen, mit einem Spritzenzylinder, der ein proximales und ein distales Ende aufweist, mit einem im Spritzenzylinder angeordneten Kolbenstopfen, und mit einem Rückanschlag, der den Kolbenstopfen gegen unbeabsichtigtes Herausziehen aus dem Spritzenzylinder sichert, wobei der Rückanschlag zumindest zwei krallenartige Fanghaken aufweist, die außerhalb des Spritzenzylinders an zueinander versetzten Umfangspositionen angeordnet sind.

Die Erfindung betrifft ferner einen Rückanschlag für eine solche Spritze, mit einem Befestigungselement zum Befestigen des Rückanschlags am proximalen Ende eines Spritzenzylinders und mit zumindest zwei krallenartigen Fanghaken, die außerhalb des Spritzenzylinders an zueinander versetzten Umfangspositionen angeordnet sind.

In der pharmazeutischen Industrie gibt es eine Reihe von Medikamenten, die vom pharmazeutischen Hersteller unter sterilen Bedingungen in einen distal mit einer dichtenden Kappe verschlossenen Spritzenzylinder abgefüllt werden. Anschließend wird der Spritzenzylinder am proximalen Ende mit einem sterilen Kolbenstopfen, häufig aus gummielastischem Material, verschlossen. Daran anschließend wird üblicherweise die Kolbenstange der Spritze in ein Innengewinde am Kolbenstopfen eingeschraubt, die Spritze steril verpackt und endkonfektioniert. Ein besonderer Vorteil dieser Vorgehensweise ist, dass das Medikament auf diese Weise bis zur eigentlichen Applikation steril gehalten ist, was beispielsweise dann, wenn das Medikament erst unmittelbar vor der Applikation in die Spritze aufgezogen wird, nicht immer gewährleistet ist.

Es ist klar, dass diese Sterilität nicht mehr gewahrt ist, wenn der Kolbenstopfen bei oder vor der Applikation des Medikaments versehentlich aus dem Spritzenzylinder herausgezogen wird. Bei sehr teuren Medikamenten ist der finanzielle Verlust entsprechend hoch. Handelt es sich zudem um ein hochtoxisches Medikament, beispielsweise ein Krebsmittel, führt das versehentliche Herausziehen des Kolbenstopfens auch zu einer Gefährdung des Arztes und/oder Patienten.

Das unbeabsichtigte Herausziehen des Kolbenstopfen aus dem Spritzenzylinder kann durch einen Rückanschlag (teilweise auch Backstop oder Kolbenbremse genannt) verhindert werden. Im Stand der Technik ist eine Vielzahl derartiger Rückanschläge bekannt. So zeigt WO 94/13339 beispielsweise einen Rückanschlag, bei dem ein krallenartiger Fanghaken in das Innere des Spritzenzylinders hineinragt und dort in eine sägezahnförmige Kante der Kolbenstange eingreift. Der Fanghaken wirkt in Verbindung mit den Sägezähnen an der Kolbenstange wie eine Sperrklinke, die ein Zurückziehen des Kolbenstopfens verhindert.

Aus WO 92/08507 ist eine Spritze bekannt, bei der eine plattenförmige Fingerauflage auf das proximale Ende des Spritzenzylinders aufgesteckt wird. Die Fingerauflage besitzt einen hülsenartigen Fortsatz, der passgenau in den Spritzenzylinder eingesetzt wird und dort mit einem Wulst am proximalen Ende des Spritzenzylinders verrastet. Der hülsenartige Fortsatz bildet einen Rückanschlag für den Kolbenstopfen, da er den lichten Innendurchmesser des Spritzenzylinders am proximalen Ende verengt.

Weitere gattungsgemäße Rückanschläge sind aus EP 0 738 517 B1 oder EP 0 764 450 B1 bekannt. Auch in diesen Fällen ist der Rückanschlag mit einer plattenförmigen Fingerauflage kombiniert. Die Fingerauflage ist so ausgebildet, dass sie seitlich auf das proximale Ende eines Spritzenzylinders aufgeschoben werden kann. Eine vorspringende Kante verengt den lichten Innendurchmesser des Spritzenzylinders und verhindert so die unbeabsichtigte Entnahme des Kolbenstopfens.

Aus WO 99/55402 ist es bekannt, krallenartige Fanghaken an der Kolbenstange selbst auszubilden. Die Fanghaken sind bei eingesteckter Kolbenstange zum proximalen Ende des Spritzenzylinders geneigt und verhindern in Verbindung mit einem im Spritzenzylinder vorgesehenen Vorsprung eine Entnahme der Kolbenstange.

Weitere Rückanschläge sind aus US 5,250,030, DE 44 34 644 C2, DE 199 29 325 A1, JP 2001-327600 A u.a. bekannt.

In US 4,946,441 ist ein Rückanschlag beschrieben, der allerdings vorrangig dazu dient, eine unbefugte Wiederverwendung der Spritze nach deren erstmaligem Gebrauch zu verhindern. Die krallenartigen Fanghaken sind dort Bestandteil einer Überwurfmutter, die am proximalen Ende des Spritzenzylinders auf ein dort ausgebildetes Außengewinde aufgeschraubt wird. Beim Aufschrauben werden die Fanghaken radial nach innen gedrückt, bis sie zur Anlage an der Kolbenstange kommen und ein Herausziehen der Kolbenstange aus der Spritze verhindern.

Unter den zahlreichen Vorschlägen für Rückanschläge befinden sich viele, die eine spezielle Ausbildung des Spritzenzylinders und/oder der Kolbenstange erfordern. All diese Lösungen sind damit nicht auf "konventionelle" Spritzen übertragbar und dementsprechend teuer. Nur wenige Lösungen, wie etwa die Rückanschläge aus EP 0 738 517 B1, können in Kombination mit herkömmlichen Spritzenzylindern und Kolbenstangen verwendet werden, so dass eine herkömmliche Spritze wahlweise und auch nachträglich mit einem Rückanschlag versehen werden kann.

Es ist eine Aufgabe der vorliegenden Erfindung, einen alternativen Rückanschlag für eine Spritze anzugeben, der bei einer Vielzahl von Spritzen, insbesondere bei Spritzen mit konventionellen Glaszylindern, anwendbar ist.

Es ist ferner eine Aufgabe der Erfindung, eine Spritze mit einem solchen Rückanschlag anzugeben.

Die genannte Aufgabe wird durch eine Spritze bzw. einen Rückanschlag der eingangs genannten Art gelöst, wobei die Fanghaken an einer plattenförmigen Fingerauflage angeordnet sind, die am proximalen Ende des Spritzenzylinders sitzt bzw. dazu ausgebildet ist, dort angeordnet zu werden.

Bei dem neuen Rückanschlag sind krallenartigen Fanghaken an einer plattenförmigen Fingerauflage angeordnet. Damit ist es möglich, den neuen Rückanschlag auch bei konventionellen Spritzen einzusetzen. Eine spezielle Ausbildung des proximalseitigen Spritzenflansches ist nicht zwingend erforderlich, wie an Hand der bevorzugten Ausführungsbeispiele nachfolgend gezeigt wird.

Der neue Rückanschlag unterscheidet sich darüber hinaus konzeptionell von allen bislang bekannten Rückanschlägen für konventionelle Spritzenzylinder. Die genannte Aufgabe ist daher vollständig gelöst.

Als zusätzlichen Vorteil ermöglicht der neue Rückanschlag die einfache Entnahme des Kolbenstopfens, wenn dies gezielt gewollt ist, indem lediglich einer der Fanghaken aufgebogen oder abgetrennt werden muss. Da eine solche Manipulation andererseits sichtbare Spuren hinterlässt, kann der Kolbenstopfen nicht später wieder unbemerkt in den Spritzenzylinder eingeführt werden. Dies gilt insbesondere, wenn der Rückanschlag insgesamt irreversibel am Spritzenzylinder befestigt wird, wie dies in einer bevorzugten Ausgestaltung vorgesehen ist. Im Gegensatz dazu ist bei den bekannten Rückanschlägen eine bewusste Entnahme des Kolbenstopfens entweder gar nicht möglich, oder aber sie ist in einer Art und Weise möglich, die keine sichtbaren Spuren hinterlässt, so dass die Gefahr besteht, dass bewusste Manipulationen an der Spritze unerkannt bleiben. Einen einfachen und gegen unerkannte Manipulationen geschützten Zugriff auf den Kolbenstopfen bietet keiner der bislang bekannten Rückanschläge.

In einer bevorzugten Ausgestaltung sind die Fanghaken frei zugänglich an der Fingerauflage angeordnet.

Frei zugänglich bedeutet in diesem Zusammenhang, dass die Fanghaken für den Bediener der Spritze ohne Weiteres erkennbar sind und der Bediener zumindest einen der Fanghaken so erreichen kann, dass er diesen aufbiegen oder abbrechen kann. Diese Ausgestaltung ist daher besonders geeignet, um die zuvor genannten Vorteile zu erreichen.

In einer weiteren Ausgestaltung sind die Fanghaken mit ihren freien Enden zum distalen Ende des Spritzenzylinders geneigt.

Durch diese Ausgestaltung wird die Haltekraft des Rückanschlags in Bezug auf den Kolbenstopfen beträchtlich erhöht. Dementsprechend ist die Gefahr, dass der Kolbenstopfen unbeabsichtigt aus dem Spritzenzylinder herausgezogen wird, weiter reduziert. Darüber hinaus ist auch das Risiko einer unerkannten Manipulation an dem Rückanschlag weiter gemindert.

In einer weiteren Ausgestaltung ist die Fingerauflage ein separates Bauteil, das am proximalen Ende des Spritzenzylinders befestigt ist.

Alternativ hierzu ist es grundsätzlich auch möglich, dass die Fingerauflage mit den Fanghaken einstückig am proximalen Ende des Spritzenzylinders ausgebildet ist. Demgegenüber besitzt die bevorzugte Ausgestaltung den Vorteil, dass der neue Rückanschlag wahlweise und insbesondere bei konventionellen Spritzenzylindern mit Rundflansch eingesetzt werden kann. Ein weiterer Vorteil liegt darin, dass der neue Rückanschlag dieser Ausgestaltung ohne Weiteres aus einem anderen Material hergestellt sein kann als der Spritzenzylinder. Damit kann insbesondere ein Rückanschlag aus Kunststoff, der hohe Haltekräfte verträgt, an Spritzenzylindern aus Glas verwendet werden.

In einer weiteren Ausgestaltung ist die Fingerauflage mit einer zylinderschalenförmigen Wand verbunden, die den Spritzenzylinder am proximalen Ende passgenau umgreift.

Der zylinderschalenförmige Wand (also ein hülsenartiger Fortsatz) ermöglicht eine sehr stabile Befestigung des neuen Rückanschlags an konventionellen Spritzenzylindern. Damit ist diese Ausgestaltung für die Lösung der oben genannten Aufgabe besonders gut geeignet.

In einer weiteren Ausgestaltung weist die Wand eine seitliche Öffnung auf, die ein seitliches Aufschieben auf den Spritzenzylinder ermöglicht.

In dieser Ausgestaltung kann der neue Rückanschlag besonders einfach und rationell in bestehende Fertigungsprozesse integriert werden. Die Verwendung des neuen Rückanschlags an konventionellen Spritzenzylindern wird dadurch noch weiter erleichtert.

In einer weiteren Ausgestaltung ist die Fingerauflage am proximalen Ende des Spritzenzylinders unlösbar befestigt. In einem bevorzugten Ausführungsbeispiel greift dabei ein Sprengring in eine an der Wand ausgebildete Nut ein.

Diese Ausgestaltung verbessert den Manipulationsschutz und macht damit besonders vorteilhaften Gebrauch von den bereits weiter oben angesprochenen Möglichkeiten. Die Verwendung eines Sprengrings in der beschriebenen Art und Weise ermöglicht eine besonders rationelle und kostengünstige Montage.

In einer weiteren Ausgestaltung sind die Fanghaken einstückig mit der Fingerauflage verbunden.

Durch diese Ausgestaltung lassen sich die Herstellungskosten der neuen Spritze bzw. des neuen Rückanschlags weiter minimieren. Insbesondere ist es in dieser Ausgestaltung möglich, die Fanghaken und die Fingerauflage in einem einzigen Spritzgießschritt herzustellen. Die damit verbundene Reduzierung der Teilezahl ermöglicht zudem eine einfache Montage der Spritze.

In einer weiteren Ausgestaltung enden die freien Enden der Fanghaken oberhalb einer Abschlussebene, die vom proximalen Ende des Spritzenzylinders definiert wird.

Diese Ausgestaltung besitzt den Vorteil, dass der lichte Innenraum des Spritzenzylinders durch den neuen Rückanschlag nicht verkleinert wird. Wenn gewünscht, steht damit der gesamte Innenraum des Spritzenzylinders zur Aufnahme des Medikaments zur Verfügung. Darüber hinaus erleichtert diese Ausgestaltung eine bewusste Manipulation an den Fanghaken.

In einer weiteren Ausgestaltung enden die freien Enden der Fanghaken auf oder außerhalb einer Kreislinie, die dem lichten Innendurchmesser des Spritzenzylinders entspricht.

Auch diese Ausgestaltung trägt dazu bei, dass eine Entnahme des Kolbenstopfens wirkungsvoll verhindert wird, ohne das Innenvolumen des Spritzenzylinders zu reduzieren. Selbst wenn die freien Enden der Fanghaken außerhalb der genannten Kreislinie enden, ist ein wirkungsvoller Schutz gegen die Entnahme des Kolbenstopfens gegeben, da die Kolbenstopfen in dem Spritzenzylinder in aller Regel komprimiert sind, um eine gute Abdichtung an den Innenwänden des Spritzenzylinders zu erreichen. Sobald der Kolbenstopfen den Spritzenzylinder verlässt, dehnt er sich aus und gelangt dadurch in Anlage an die Fanghaken. Diese bevorzugte Ausgestaltung besitzt den Vorteil, dass die Kolbenstange beim Betätigen der Spritze nicht an den Fanghaken anliegt, d.h. die Spritze kann unbeeinflusst von dem Rückanschlag betätigt werden. Hierdurch lässt sich eine feinere Dosierung erreichen.

Es versteht sich, dass die vorstehend genannten und die nachstehend noch zu erläuternden Merkmale nicht nur in der jeweils angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in der Zeichnung dargestellt und werden in der nachfolgenden Beschreibung näher erläutert. Es zeigen:
- Fig. 1: ein Ausführungsbeispiel der neuen Spritze in einer Seitenansicht,
- Fig. 2: den Spritzenzylinder und den Rückanschlag des Ausführungsbeispiels aus Fig. 2 in einer perspektivischen Darstellung,
- Fig. 3: den Spritzenzylinder und den Rückanschlag gemäß Fig. 2 in einer Querschnittsansicht, und
- Fig. 4: die Spritze aus Fig. 1 ohne den Spritzenzylinder zur Erläuterung der Funktionsweise des neuen Rückanschlags.

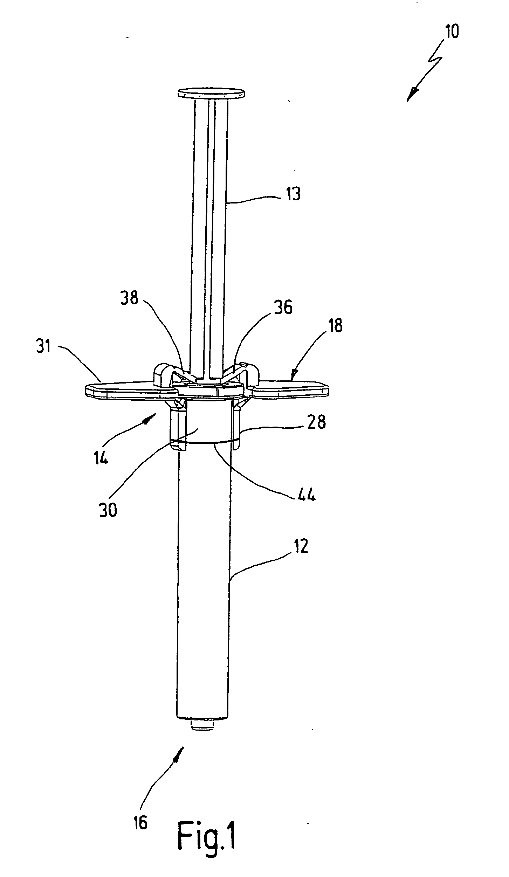

In Fig. 1 ist ein Ausführungsbeispiel der neuen Spritze in seiner Gesamtheit mit der Bezugsziffer 10 bezeichnet.

Die Spritze 10 besitzt in an sich bekannter Weise einen Spritzenzylinder 12 und eine Kolbenstange 13, die am proximalen Ende 14 des Spritzenzylinders 12 eingesteckt ist. Das distale Ende 16 des Spritzenzylinders 12 besitzt einen Anschlüsse beispielsweise einen Luer-Lock-Anschluss, zum Anbringen einer hier nicht gezeigten Nadel.

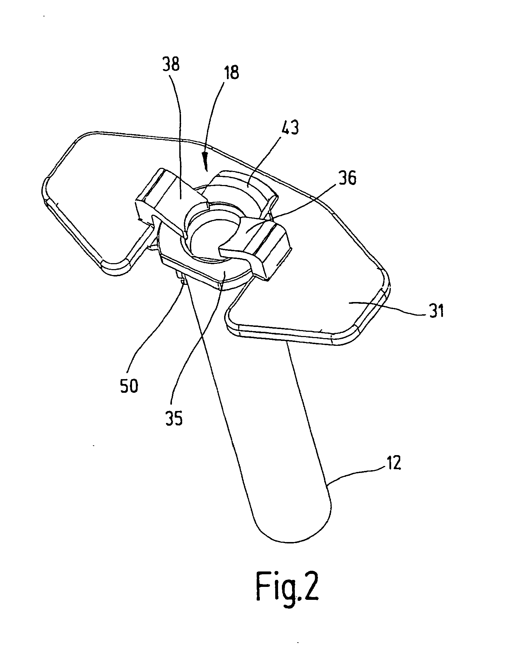

Für die weitere Beschreibung wird ergänzend auch auf die Figuren 2 bis 4 Bezug genommen, in denen gleiche Bezugszeichen jeweils dieselben Elemente bezeichnen.

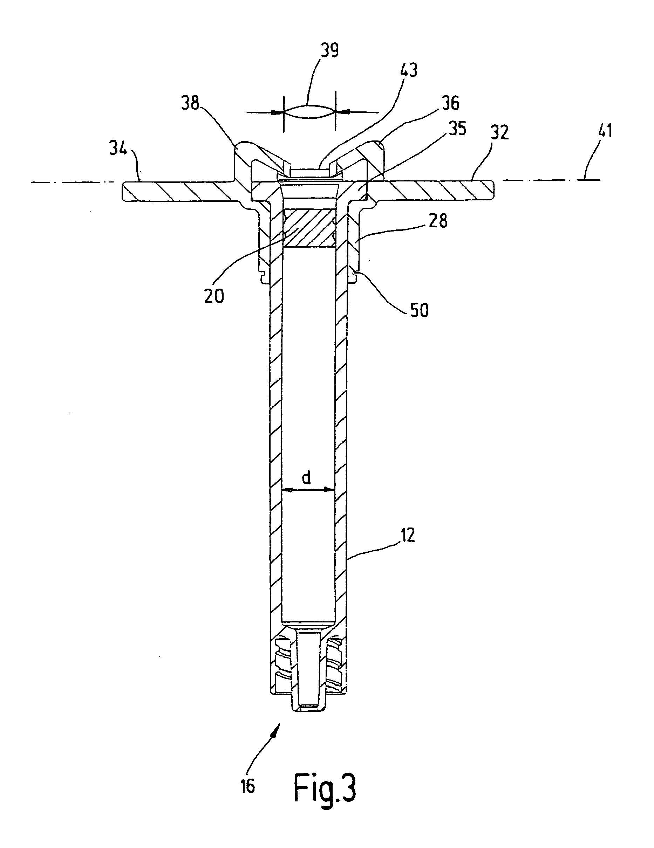

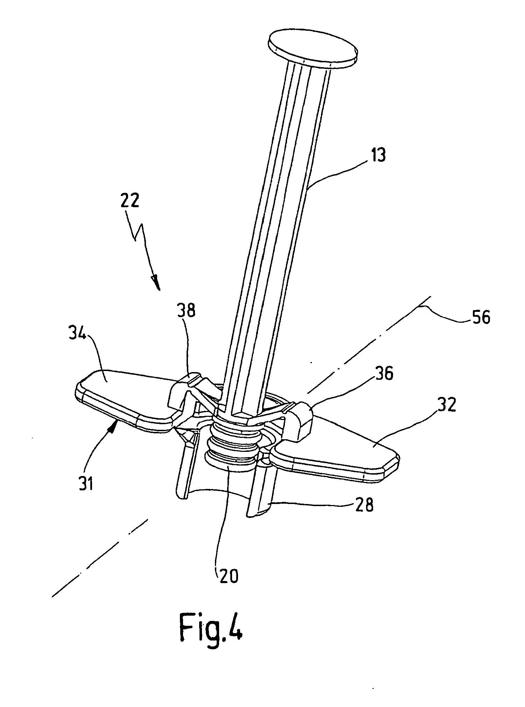

Mit Bezugsziffer 18 ist ein Ausführungsbeispiel des neuen Rückanschlags bezeichnet. Der Rückanschlag 18 dient dazu, eine unbeabsichtigte Entnahme des Kolbenstopfens 20 (siehe Fig. 3, Fig. 4) aus dem Spritzenzylinder 12 zu verhindern. Zusammen mit dem Kolbenstopfen 20 bildet der Rückanschlag 18 ein proximales Verschlusselement, das es ermöglicht, die Spritze 10 im vorgefüllten Zustand an Endabnehmer auszuliefern. Es versteht sich, dass das distale Ende 16 in diesem Fall auch mit einem Verschlusselement abgedichtet ist, beispielsweise mit einer so genannten Tip-Cap oder einer Nadel mit Nadelschutzkappe.

Der Rückanschlag 18 besitzt im gezeigten Ausführungsbeispiel eine zylinderschalenförmige Wand 28, die das proximale Ende des Spritzenzylinders 12 passgenau umgreift. Die Wand 28 hat einen Öffnungsbereich 30, der es ermöglicht, den Rückanschlag 18 seitlich auf das proximale Ende 14 des Spritzenzylinders 12 aufzuschieben.

Des Weiteren besitzt der Rückanschlag 18 eine Fingerauflage 31 mit zwei plattenförmigen Bereichen 32, 34, die sich auf zwei gegenüberliegenden Seiten des Spritzenzylinders 12 quer zu der Wand 28 erstrecken. Die Plattenbereiche 32, 34 schließen bündig mit dem proximalen Ende des Spritzenzylinders 12 ab. Mit anderen Worten umgreifen die beiden Plattenbereiche 32, 34 den proximalen Abschlussflansch 35 des Spritzenzylinders 12 bündig, während die zylinderschalenförmige Wand 28 unterhalb des proximalen Abschlussflansches 35 am Spritzenzylinder 12 anliegt.

Seitlich vom Spritzenzylinder 12 ist auf den Plattenbereichen 32, 34 jeweils ein krallenartiger Fanghaken 36, 38 angeordnet. Im gezeigten und derzeit bevorzugten Ausführungsbeispiel sind die Fanghaken 36, 38, die Fingerauflage 31 mit den Plattenbereichen 32, 34 und die Wand 28 einstückig miteinander ausgebildet. Abweichend hiervon könnte der Rückanschlag 18 jedoch grundsätzlich auch aus mehreren zusammengefügten Teilen bestehen.

Die Fanghaken 36, 38 sind jeweils etwa L-förmig ausgebildet, wobei ein kurzer Schenkel des L senkrecht von dem jeweiligen Plattenbereich 32, 34 nach oben steht, während ein längerer Schenkel mit einer Neigung zum distalen Ende 16 des Spritzenzylinders 12 radial nach innen zeigt. Die freien Enden der Fanghaken 36, 38 weisen damit radial aufeinander zu. Sie befinden sich im vorliegenden Ausführungsbeispiel genau auf oder geringfügig außerhalb einer Kreislinie 39, deren Durchmesser dem lichten Innendurchmesser d des Spritzenzylinders 12 entspricht. In anderen Ausführungsbeispielen können die freien Enden der Fanghaken 36, 38 jedoch auch in die projizierte Kreislinie 39 hineinragen.

Des Weiteren liegen die freien Enden der Fanghaken 36, 38 in diesem Ausführungsbeispiel oberhalb einer Abschlussebene 41, die vom proximalen Ende 14 des Spritzenzylinders 12 definiert wird. Abweichend hiervon ist es grundsätzlich jedoch auch möglich, dass die freien Enden der Fanghaken 36, 38 unterhalb der Ebene 41 enden und somit in den Spritzenzylinder 12 hineinragen.

Mit der Bezugsziffer 43 (vgl. Fig. 2) ist ein Kantenstück bezeichnet, das einstückig mit der Fingerauflage 31 des Rückanschlags 18 ausgebildet ist. Das Kantenstück 43 befindet sich am Außenumfang des Spritzenzylinders 12 etwa mittig zwischen den beiden Fanghaken 36, 38. Seine Abmessungen sind so gewählt, dass es auf dem proximalen Abschlussflansch 35 des Spritzenzylinders 12 aufliegt, ohne in den lichten Innendurchmesser des Spritzenzylinders 12 hineinzuragen. Das Kantenstück 43 sichert den Rückanschlag 18 damit gegen ein Durchrutschen zum distalen Ende 16 des Spritzenzylinders 12.

Mit der Bezugsziffer 44 ist in Fig. 1 ein Ring bezeichnet, der in einer Nut 50 verläuft, die am unteren (distalen) Ende der Wand 28 ausgebildet ist. Der Ring 44 verhindert ein seitliches Abziehen des Rückanschlags 18 vom Spritzenzylinder 12, so dass bewusste Manipulationen verhindert oder zumindest erkennbar gemacht sind. In besonders vorteilhafter Weise kann der Ring 44 Bestandteil eines Originalitätsverschlusses sein, der in seiner Gesamtheit Gegenstand einer parallelen Anmeldung der vorliegenden Anmelderin ist. In anderen Ausführungsbeispielen kann der Ring 44 grundsätzlich jedoch auch entfallen, oder der Rückanschlag 18 wird auf andere Weise, beispielsweise durch Kleben, Schrumpfen oder Schweißen, am Spritzenzylinder 12 irreversibel befestigt.

Fig. 4 verdeutlicht die grundsätzliche Funktionsweise des Rückanschlags 18. Die freien Enden der Fanghaken 36, 38 können sich auf Grund der beschriebenen Anordnung um eine Achse 56 drehen, die aus Gründen der Übersichtlichkeit nur für den Fanghaken 36 dargestellt ist. Die Achse 56 liegt oberhalb der Ebene 41 und außerhalb des lichten Innendurchmessers d des Spritzenzylinders 12. Wird der Kolbenstopfen 20 aus dem Spritzenzylinder 12 herausgezogen, gerät er zwischen die beiden freien Enden der Fanghaken 36, 38 und gelangt dort zur Anlage. Ein weiteres Herausziehen des Kolbenstopfens 20 führt dazu, dass die freien Enden eine Drehbewegung um die entsprechende Achse 56 durchführen. Der lichte Abstand zwischen den beiden Enden wird reduziert, der Kolbenstopfen 20 also fester geklemmt. Eine vollständige Entnahme des Kolbenstopfens 20 ist auf Grund der geometrischen Abmessungen der Fanghaken 36, 38 ohne Zerstörung der Fanghaken nicht möglich.

In dem in den Figuren dargestellten Ausführungsbeispiel ist der Rückanschlag 18 mit der Fingerauflage 31 und den Fanghaken 36, 38 so ausgebildet, dass er auf konventionelle Spritzenzylinder 12 mit Rundflansch aufgesetzt werden kann. Alternativ hierzu ist es jedoch auch möglich, den Rückanschlag 18 so auszubilden, dass er bei Spritzenzylindern verwendet werden kann, die einen anderen Flansch besitzen. Darüber hinaus ist es grundsätzlich auch möglich, das Grundprinzip des vorliegenden Rückanschlags 18 bei Spritzenzylindern anzuwenden, die bereits von sich aus eine Fingerauflage 31 besitzen. Auch in diesem Fall können entsprechende Fanghaken 36, 38 oberhalb der Fingerauflage 31 angeordnet werden, sei es einstückig oder durch ein ergänzendes Montageteil. Außerdem ist die Erfindung nicht auf Ausführungsbeispiele mit zwei Fanghaken beschränkt. Es können auch drei oder mehr radial aufeinander zuweisende Fanghaken verwendet werden.

## Patentansprüche

1. Spritze, insbesondere für medizinische Anwendungen, mit einem Spritzenzylinder (12), der ein proximales (14) und ein distales (16) Ende aufweist, mit einem im Spritzenzylinder (12) angeordneten Kolbenstopfen (20), und mit einem Rückanschlag (18), der den Kolbenstopfen (20) gegen unbeabsichtigtes Herausziehen aus dem Spritzenzylinder (12) sichert, **dadurch gekennzeichnet, dass** der Rückanschlag (18) zumindest zwei krallenartige Fanghaken (36, 38) aufweist, die außerhalb des Spritzenzylinders (12) an zueinander versetzten Umfangspositionen angeordnet sind, und dass die Fanghaken (36, 38) an einer plattenförmigen Fingerauflage (31) angeordnet sind, die am proximalen Ende (14) des Spritzenzylinders (12) sitzt.

2. Spritze nach Anspruch 1, **dadurch gekennzeichnet, dass** die Fanghaken (36, 38) an der Fingerauflage (31) frei zugänglich angeordnet sind.

3. Spritze nach Anspruch 1 oder 2, **dadurch gekennzeichnet, dass** die Fanghaken (36, 38) mit ihren freien Enden zum distalen Ende (16) des Spritzenzylinders (12) geneigt sind.

4. Spritze nach einem der Ansprüche 1 bis 3, **dadurch gekennzeichnet, dass** die Fingerauflage (31) ein separates Bauteil ist, das am proximalen Ende (14) des Spritzenzylinders (12) befestigt ist.

5. Spritze nach einem der Ansprüche 1 bis 4, **dadurch gekennzeichnet, dass** die Fingerauflage (31) mit einer zylinderschalenförmigen Wand (28) verbunden ist, die den Spritzenzylinder (12) am proximalen Ende (14) passgenau umgreift.

6. Spritze nach Anspruch 5, **dadurch gekennzeichnet, dass** die Wand (28) eine seitliche Öffnung (30) aufweist, die ein seitliches Aufschieben auf den Spritzenzylinder (12) ermöglicht.

7. Spritze nach einem der Ansprüche 1 bis 6, **dadurch gekennzeichnet, dass** die Fingerauflage (31) am proximalen Ende (14) des Spritzenzylinders (12) unlösbar befestigt ist, und zwar vorzugsweise mit einem Sprengring (44), der in eine an der Wand (28) ausgebildete Nut (50) eingreift.

8. Spritze nach einem der Ansprüche 1 bis 7, **dadurch gekennzeichnet, dass** die Fanghaken (36, 38) einstückig mit der Fingerauflage (31) verbunden sind.

9. Spritze nach einem der Ansprüche 1 bis 8, **dadurch gekennzeichnet, dass** die freien Enden der Fanghaken (36, 38) oberhalb einer Abschlussebene (41) enden, die vom proximalen Ende (14) des Spritzenzylinders (12) definiert wird.

10. Spritze nach einem der Ansprüche 1 bis 9, **dadurch gekennzeichnet, dass** die freien Enden der Fanghaken (36, 38) auf oder außerhalb einer Kreislinie (39) enden, die dem lichten Innendurchmesser (d) des Spritzenzylinders (12) entspricht.

11. Rückanschlag für eine Spritze nach einem der Ansprüche 1 bis 10, mit einem Befestigungselement (28) zum Befestigen des Rückanschlages (18) am proximalen Ende (14) eines Spritzenzylinders (12) **dadurch gekennzeichnet, dass** dieser zumindest zwei krallenartigen Fanghaken (36, 38) aufweist, die außerhalb des Spritzenzylinders (12) an zueinander versetzten Umfangspositionen angeordnet sind, und dass die Fanghaken (36, 38) an einer plattenförmigen Fingerauflage (31) angeordnet sind, die dazu ausgebildet ist, an dem proximalen Ende (14) des Spritzenzylinders (12) angeordnet zu werden.

## Claims

1. Syringe, in particular for medical applications, comprising a syringe barrel (12) having a proximal end (14) and a distal end (16), comprising a plunger stopper (20) arranged in the syringe barrel (12), and comprising a backstop (18) which secures the plunger stopper (20) against inadvertent withdrawal from the syringe barrel (12), **characterized in that** the backstop (18) has at least two claw-like catch hooks (36, 38) arranged outside the syringe barrel (12) at mutually offset circumferential positions, and the catch hooks (36, 38) are arranged on a plate-shaped finger support (31) sitting at the proximal end (14) of the syringe barrel (12).

2. Syringe according to Claim 1, **characterized in that** the catch hooks (36, 38) are arranged in a freely accessible manner on the finger support (31).

3. Syringe according to Claim 1 or 2, **characterized in that** the catch hooks (36, 38) are inclined with their free ends towards the distal end (16) of the syringe barrel (12).

4. Syringe according to one of Claims 1 to 3, **characterized in that** the finger support (31) is a separate structural part which is secured at the proximal end (14) of the syringe barrel (12).

5. Syringe according to one of Claims 1 to 4, **characterized in that** the finger support (31) is connected to a wall (28) which has the shape of a cylinder shell and which engages with a precise fit around the proximal end (14) of the syringe barrel (12).

6. Syringe according to Claim 5, **characterized in that** the wall (28) has a lateral opening (30) allowing it to be pushed from the side onto the syringe barrel (12).

7. Syringe according to one of Claims 1 to 6, **characterized in that** the finger support (31) is secured in a non-releasable manner at the proximal end (14) of the syringe barrel (12), preferably with a retainer ring (44) which engages in a groove (50) formed on the wall (28).

8. Syringe according to one of Claims 1 to 7, **characterized in that** the catch hooks (36, 38) are connected integrally to the finger support (31).

9. Syringe according to one of Claims 1 to 8, **characterized in that** the free ends of the catch hooks (36, 38) finish above a terminating plane (41) defined by the proximal end (14) of the syringe barrel (12).

10. Syringe according to one of Claims 1 to 9, **characterized in that** the free ends of the catch hooks (36, 38) end on or outside a circular line (39) corresponding to the clear internal diameter (d) of the syringe barrel (12).

11. Backstop for a syringe according to one of Claims 1 to 10, comprising a securing element (28) for securing the backstop (18) at the proximal end (14) of a syringe barrel (12), **characterized in that** said syringe barrel comprises at least two claw-like catch hooks (36, 38) arranged outside the syringe barrel (12) at mutually offset circumferential positions, and the catch hooks (36, 38) are arranged on a plate-shaped finger support (31) designed to be arranged at the proximal end (14) of the syringe barrel (12).

## Revendications

1. Seringue, en particulier à usage médical, avec un corps cylindrique (12) muni d'une extrémité proximale (14) et d'une extrémité distale (16), avec un bouchon de type piston (20) agencé dans le corps cylindrique (12), et avec une butée (18) empêchant le retrait accidentel du bouchon piston (20) hors du corps cylindrique (12), **caractérisée en ce que** la butée (18) comporte au moins deux crochets d'arrêt (36, 38) en forme de griffes, qui sont disposés à l'extérieur du corps cylindrique (12) en des emplacements décalés l'un par rapport à l'autre sur la périphérie, et **en ce que** les crochets d'arrêt (36, 38) sont disposés sur un support de doigt (31) en forme de plaque, qui est situé à l'extrémité proximale (14) du corps cylindrique (12).

2. Seringue selon la revendication 1, **caractérisée en ce que** les crochets d'arrêt (36, 38) sont disposés de manière librement accessible sur le support de doigt (31).

3. Seringue selon la revendication 1 ou 2, **caractérisée en ce que** les crochets d'arrêt (36, 38) sont inclinés avec leur extrémité libre vers l'extrémité distale (16) du corps cylindrique (12).

4. Seringue selon l'une quelconque des revendications 1 à 3, **caractérisée en ce que** le support de doigt (31) est un élément séparé, qui est fixé au niveau de l'extrémité proximale (14) du corps cylindrique (12).

5. Seringue selon l'une quelconque des revendications 1 à 4, **caractérisée en ce que** le support de doigt (31) est assemblé à une paroi (28) en forme de coque cylindrique, qui enserre de manière ajustée le corps cylindrique (12) au niveau de l'extrémité proximale (14).

6. Seringue selon la revendication 5, **caractérisée en ce que** la paroi (28) comporte une ouverture (30) latérale, qui permet un emmanchement latéral sur le corps cylindrique (12).

7. Seringue selon l'une quelconque des revendications 1 à 6, **caractérisée en ce que** le support de doigt (31) est fixé de manière inamovible sur l'extrémité proximale (14) du corps cylindrique (12), à savoir de préférence avec un circlips (44), qui s'engage dans une rainure (50) ménagée dans la paroi (28).

8. Seringue selon l'une quelconque des revendications 1 à 7, **caractérisée en ce que** les crochets d'arrêt (36, 38) sont assemblés d'un seul tenant au support de doigt (31).

9. Seringue selon l'une quelconque des revendications 1 à 8, **caractérisée en ce que** les extrémités libres des crochets d'arrêt (36, 38) se terminent au-dessus d'un plan de fermeture (41), qui est défini par l'extrémité proximale (14) du corps cylindrique (12).

10. Seringue selon l'une quelconque des revendications 1 à 9, **caractérisée en ce que** les extrémités libres des crochets d'arrêt (36, 38) se terminent sur ou en dehors d'une ligne de cercle (39), qui correspond au diamètre intérieur (d) du corps cylindrique (12).

11. Butée pour une seringue selon l'une quelconque des revendications 1 à 10, comportant un élément de fixation (28) pour fixer la butée (18) sur l'extrémité proximale (14) d'un corps cylindrique (12), **caractérisée en ce que** ladite butée comporte au moins deux crochets d'arrêt (36, 38) en forme de griffes, qui sont disposés à l'extérieur du corps cylindrique (12) en des emplacements décalés l'un par rapport à l'autre sur la périphérie, et **en ce que** les crochets d'arrêt (36, 38) sont disposés sur un support de doigt (31) en forme de plaque, qui est réalisé pour être monté au niveau de l'extrémité proximale (14) du corps cylindrique (12).
